# EUROPEAN PATENT APPLICATION

(11) **EP 2 977 386 A1**
(43) Date of publication of application: **27.01.2016**
(21) Application number: 15181968.7
(22) Date of filing: 30.08.2013
(51) Int. Cl.: C07K 16/28, C12N 15/10, C40B 40/08

(54) **METHOD FOR PRODUCING ANTIBODY MOLECULES HAVING INTER-SPECIES, INTRA-TARGET CROSS-REACTIVITY**

(30) Priority: 31.08.2012 US 201261695664 P
(62) Divisional of application: 13753659.5
(71) Applicant: ARGEN-X NV, 4811 AH Breda (NL)
(72) Inventor: DE HAARD, Johannes Joseph Wilhelmus, 4811 AH Breda (NL); BLANCHETOT, Christophe Frederic Jerome, 4811 AH Breda (NL); VAN DER WONING, Sebastian Paul, 4811 AH Breda (NL)
(74) Representative: Boult Wade Tennant

(57) **Abstract**

A method is disclosed for producing an antibody molecule having inter-species, intra-target cross-reactivity. The method comprises the steps of creating a first immune library for a target antigen from a first species, for example man; combining variable regions from the first immune library in a chain shuffling protocol, to produce a second immune library; and screening the second immune library for affinity to both the target antigen and a corresponding antigen from a second species. The second species is different from the first species. By using a suitable test animal as the second species, the method paves the way to convenient testing of the antibody molecule.

SEQUENCE LISTING

<110>  Argen-X NV

<120>  METHOD FOR PRODUCING ANTIBODY MOLECULES HAVING INTER-SPECIES,
       INTRA-TARGET CROSS-REACTIVITY

<130>  NLW/P124542EP01

<150>  US61/695,664
<151>  2012-08-31

<160>  14

<170>  PatentIn version 3.5

<210>  1
<211>  117
<212>  PRT
<213>  Artificial

<220>
<223>  VH Sequence

<400>  1

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Ser
            20                  25                  30

Trp Met Tyr Trp Val Arg Gln Ala Pro Gly Lys Gly Leu Glu Phe Val
        35                  40                  45

Ser Val Ile Asn Thr Arg Ser Gly Ile Thr His Tyr Val Glu Ser Val
    50                  55                  60

Lys Gly Arg Phe Thr Ile Ser Thr Asp Asn Ala Lys Asn Thr Leu Phe
65                  70                  75                  80

Leu Gln Met Asn Ser Leu Lys Ser Glu Asp Thr Ala Val Tyr Tyr Cys
                85                  90                  95

Ala Thr Arg Ile Gln Gly Leu Ile Asp Tyr Trp Gly Gln Gly Thr Gln
            100                 105                 110

Val Thr Val Ser Ser
        115

<210>  2
<211>  110
<212>  PRT
<213>  Artificial

<220>
<223>  VK Sequence

<400>  2

Leu Asp Ile Val Met Thr Gln Ser Pro Ser Ser Leu Ser Val Ser Leu
1               5                   10                  15

Gly Asp Arg Val Thr Ile Thr Cys Gln Ala Ser Gln Ser Ile Gly Ser
            20                  25                  30

Val Asn Ala Leu Ala Trp Tyr Gln Gln Lys Pro Gly Gln Ser Leu Lys
        35                  40                  45

Leu Leu Ile His Gly Ala Ser Arg Leu Gln Thr Gly Val Pro Ser Arg
    50                  55                  60

Phe Ser Gly Ser Gly Ser Gly Thr Ser Phe Thr Leu Ala Ile Ser Gly
65                  70                  75                  80

Val Glu Ala Glu Asp Leu Ala Thr Tyr Tyr Cys Leu Gln Asp Tyr Ala
                85                  90                  95

Trp Pro Leu Thr Phe Gly Gln Gly Thr Lys Val Glu Leu Lys
            100                 105                 110

<210>  3
<211>  108
<212>  PRT
<213>  Artificial

<220>
<223>  VK Sequence

<400>  3

Leu Asp Ile Val Met Thr Gln Ser Pro Ser Ser Leu Ser Ala Ser Leu
1               5                   10                  15

Gly Asp Arg Val Thr Ile Thr Cys Gln Ala Thr Gln Ser Ile Ser Thr
            20                  25                  30

Phe Leu Ser Trp Tyr Gln Gln Lys Pro Gly Gln Thr Pro Lys Leu Leu
        35                  40                  45

Val Tyr Gly Ala Ser Arg Leu Gln Thr Gly Val Pro Ser Arg Phe Ser
    50                  55                  60

Gly Thr Gly Ser Gly Thr Lys Phe Thr Leu Thr Ile Ser Gly Val Glu
65                  70                  75                  80

Ala Glu Asp Leu Ala Ser Tyr Phe Cys Leu Gln Asp Tyr Ser Trp Pro
                85                  90                  95

Leu Thr Phe Gly Gln Gly Thr Lys Val Glu Leu Lys
            100                 105

<210>  4
<211>  108
<212>  PRT
<213>  Artificial

<220>
<223>  VK Sequence

<400>  4

Leu Asp Ile Val Met Thr Gln Ser Pro Ser Ser Leu Ser Ala Ser Leu
1               5                   10                  15

Gly Asp Arg Val Thr Ile Thr Cys Gln Ala Ser Gln Ser Ile Arg Ser
            20                  25                  30

Phe Ile Asp Trp Tyr Gln Gln Lys Pro Gly Gln Ala Pro Lys Leu Leu
        35                  40                  45

Ile Tyr Gly Thr Ser Arg Leu Glu Thr Gly Val Pro Ser Arg Phe Ser
    50                  55                  60

Gly Thr Gly Ser Gly Thr Ser Phe Thr Leu Thr Ile Ser Gly Val Glu
65                  70                  75                  80

Ala Glu Asp Leu Ala Thr Tyr Tyr Cys Leu Gln Asp Tyr Ser Trp Pro
                85                  90                  95

Leu Thr Phe Gly Gln Gly Thr Lys Val Glu Leu Lys
            100                 105

<210>  5
<211>  108
<212>  PRT
<213>  Artificial

<220>
<223>  VK Sequence

<400>  5

Leu Asp Ile Gln Met Thr Gln Ser Pro Ser Ser Leu Ser Ala Ser Leu
1               5                   10                  15

Gly Asp Arg Val Thr Ile Thr Cys Gln Ala Ser Gln Ser Ile Ser Ser
            20                  25                  30

Met Leu Ala Trp Tyr Gln Gln Lys Pro Gly Gln Ala Pro Asn Leu Leu
        35                  40                  45

Ile Tyr Gly Ala Ser Arg Leu Gln Thr Gly Val Pro Ser Arg Phe Ser
    50                  55                  60

Gly Ser Arg Ser Gly Thr Ser Phe Thr Leu Thr Ile Ser Gly Val Glu
65                  70                  75                  80

Ala Glu Asp Leu Ala Thr Tyr Tyr Cys Leu Gln Asp Tyr Ser Trp Pro
                85                  90                  95

Leu Thr Phe Gly Gln Gly Thr Lys Val Glu Leu Lys
            100                 105

<210>  6
<211>  108
<212>  PRT
<213>  Artificial

<220>
<223>  VK Sequence

<400>  6

Leu Asp Ile Gln Met Thr Gln Ser Pro Ser Ser Leu Ser Ala Ser Leu
1               5                   10                  15

Gly Asp Arg Val Thr Ile Thr Cys Gln Ala Ser Gln Ser Ile Ser Ser
            20                  25                  30

Gln Leu Ala Trp Tyr Gln Gln Lys Pro Gly Gln Ala Pro Lys Leu Leu
        35                  40                  45

Ile Tyr Gly Ala Ser Arg Leu Gln Thr Gly Val Pro Ser Arg Phe Ser
    50                  55                  60

Gly Ser Gly Ser Gly Thr Ser Phe Thr Leu Thr Ile Ser Gly Val Glu
65                  70                  75                  80

Ala Glu Asp Leu Ala Thr Tyr Tyr Cys Leu Gln Asp Tyr Ser Trp Pro
                85                  90                  95

Leu Thr Phe Gly Gln Gly Thr Lys Val Glu Leu Lys
            100                 105

<210>  7
<211>  108
<212>  PRT
<213>  Artificial

<220>
<223>  VK Sequence

<400>  7

Leu Asp Ile Gln Met Thr Gln Ser Pro Ser Ser Leu Ser Ala Ser Leu
1               5                   10                  15

Gly Asp Arg Val Thr Ile Thr Cys Gln Ala Ser Gln Ser Ile Ser Ser
            20                  25                  30

Leu Leu Ala Trp Tyr Gln Gln Lys Pro Gly Gln Ala Pro Lys Leu Leu
        35                  40                  45

Ile Tyr Gly Ala Ser Arg Leu Gln Thr Gly Val Pro Ser Arg Phe Ser
    50                  55                  60

Gly Ser Gly Ser Gly Thr Ser Phe Thr Leu Thr Ile Ser Gly Val Glu
65                  70                  75                  80

Ala Glu Asp Leu Ala Thr Tyr Tyr Cys Leu Gln Asp Tyr Ser Trp Pro
                85                  90                  95

Leu Thr Phe Gly Gln Gly Thr Lys Val Glu Leu Lys
            100                 105


<210>  8
<211>  108
<212>  PRT
<213>  Artificial

<220>
<223>  VK Sequence

<400>  8

Leu Asp Ile Val Met Thr Gln Ser Pro Ser Ser Leu Ser Ala Ser Leu
1               5                   10                  15

Gly Asp Arg Val Thr Val Thr Cys Gln Ala Ser Gln Ser Ile Arg Ser
            20                  25                  30

Ser Leu Asn Trp Tyr Gln Gln Lys Pro Gly Gln Ala Pro Lys Leu Leu
        35                  40                  45

Ile Tyr Gly Ala Ser Arg Leu Arg Ile Gly Val Pro Ser Arg Phe Ser
    50                  55                  60

Gly Ser Ala Ser Gly Thr Ser Phe Thr Leu Thr Ile Ser Gly Val Glu
65                  70                  75                  80

Ser Glu Asp Leu Ala Thr Tyr Tyr Cys Leu Gln Asp Tyr Thr Trp Pro
                85                  90                  95

Leu Ser Phe Gly Ser Gly Thr Arg Leu Glu Ile Lys
            100                 105

<210>  9
<211>  108
<212>  PRT
<213>  Artificial

<220>
<223>  VK Sequence

<400>  9

Leu Asp Ile Gln Met Thr Gln Ser Pro Ser Ser Leu Ser Ala Ser Leu
1               5                   10                  15

Gly Asp Arg Val Thr Val Thr Cys Gln Ala Ser Gln Ser Ile Arg Ser
            20                  25                  30

Ser Leu Asn Trp Tyr Gln Gln Lys Pro Gly Gln Ala Pro Lys Leu Leu
        35                  40                  45

Ile Tyr Gly Ala Ser Arg Leu Arg Ile Gly Val Pro Ser Arg Phe Ser
    50                  55                  60

Gly Ser Ala Ser Gly Thr Ser Phe Thr Leu Thr Ile Ser Gly Val Glu
65                  70                  75                  80

Ala Glu Asp Leu Ala Thr Tyr Tyr Cys Leu Gln Glu Tyr Ala Trp Pro
                85                  90                  95

Leu Thr Phe Gly Gln Gly Thr Lys Val Glu Leu Lys
            100                 105

<210>  10
<211>  108
<212>  PRT
<213>  Artificial

<220>
<223>  VK Sequence

<400>  10

Leu Asp Ile Gln Met Thr Gln Ser Pro Ser Ser Met Ser Ala Ser Leu
1               5                   10                  15

Gly Asp Ser Val Thr Ile Ser Cys Gln Ala Ser Glu Asn Ile Gly Thr
            20                  25                  30

Gln Leu Phe Trp Tyr Gln Gln Asn Ala Gly Gln Pro Pro Lys Leu Leu
        35                  40                  45

Ile Tyr Gly Ala Ser Arg Leu Gln Thr Gly Val Pro Ser Arg Phe Ser
    50                  55                  60

Gly Ser Gly Ser Gly Thr Thr Phe Thr Leu Thr Ile Ser Gly Val Glu
65                  70                  75                  80

Thr Gly Asp Leu Ala Thr Tyr Tyr Cys Leu Gln Asp Tyr Ser Trp Pro
                85                  90                  95

Leu Thr Phe Gly Gln Gly Thr Lys Val Glu Leu Lys
            100                 105

<210>  11
<211>  108
<212>  PRT
<213>  Artificial

<220>
<223>  VL Sequence

<400>  11

Leu Ser Tyr Glu Leu Thr Gln Pro Ser Ala Val Ser Val Leu Leu Gly
1               5                   10                  15

Gln Thr Ala Lys Ile Thr Cys Gln Gly Gly Ser Leu Arg Ser Ser Tyr
            20                  25                  30

Ala His Trp Tyr Gln Gln Lys Pro Gly Gln Ala Pro Val Leu Val Ile
        35                  40                  45

Thr Glu Asp Asp Ser Arg Pro Ser Gly Ile Pro Glu Arg Phe Ser Gly
    50                  55                  60

Ser Ser Ser Gly Gly Thr Ala Ser Leu Thr Ile Ser Gly Ala Gln Ala
65                  70                  75                  80

Asp Asp Glu Ala Asp Tyr His Cys Gln Ser Ala Asp Ile Ser Gly Asn
                85                  90                  95

Pro Val Phe Gly Gly Gly Thr Gln Leu Thr Val Leu
            100                 105

<210>  12
<211>  107
<212>  PRT

<213>  Artificial

<220>
<223>  VL Sequence

<400>  12

His Ser Ala Val Thr Gln Pro Ser Ala Val Ser Val Ser Leu Gly Gln
1               5                   10                  15

Thr Ala Arg Ile Thr Cys Gln Gly Gly Asn Phe Gly Ser Tyr Tyr Ala
            20                  25                  30

Ser Trp Tyr Gln Gln Lys Pro Gly Gln Ala Pro Val Leu Val Ile Tyr
        35                  40                  45

Asp Asp Asp Arg Arg Pro Ser Gly Ile Pro Glu Arg Phe Ser Gly Ser
    50                  55                  60

Ser Ser Gly Gly Arg Ser Thr Leu Thr Ile Ser Gly Ala Gln Ser Gly
65                  70                  75                  80

Asp Glu Gly Asp Tyr Tyr Cys Gln Ser Ala Asp Leu Ser Gly Asn Pro
                85                  90                  95

Val Phe Gly Gly Gly Thr His Leu Thr Val Leu
            100                 105


<210>  13
<211>  114
<212>  PRT
<213>  Artificial

<220>
<223>  VH Sequence

<400>  13

Gln Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Asn Ser Tyr
            20                  25                  30

Glu Met Tyr Trp Val Arg Gln Ala Pro Gly Lys Gly Leu Glu Trp Val
        35                  40                  45

Ser Gly Ile Thr Ala Asp Gly Gly Tyr Thr Tyr Tyr Ala Asp Ser Val
    50                  55                  60

Lys Gly Arg Phe Thr Met Ser Arg Asp Asn Ala Lys Asn Thr Leu Tyr
65                  70                  75                  80

Leu Gln Met Asn Ser Leu Lys Pro Glu Asp Thr Ala Leu Tyr Tyr Cys
                85                  90                  95

Ala Asn Leu Trp Asn Asp Tyr Trp Gly Gln Gly Thr Gln Val Ile Val
            100                 105                 110

Ser Ser

<210>  14
<211>  114
<212>  PRT
<213>  Artificial

<220>
<223>  VH Sequence

<400>  14

Gln Leu Gln Val Val Glu Ser Gly Gly Gly Leu Val Gln Pro Gly Gly
1               5                   10                  15

Ser Leu Ser Leu Ser Cys Ala Thr Ser Gly Phe Thr Phe Ser Asn Tyr
            20                  25                  30

Glu Met Asn Trp Val Arg Gln Ala Pro Gly Lys Gly Phe Glu Trp Val
        35                  40                  45

Ser Leu Ile Asn Ser Gly Gly Ser Tyr Thr Arg Tyr Thr Asp Ser Val
    50                  55                  60

Lys Gly Arg Phe Thr Ile Ser Arg Asp Asn Ala Lys Lys Thr Leu Tyr
65                  70                  75                  80

Leu Gln Met Asn Ser Leu Lys Pro Glu Asp Thr Ala Val Tyr Tyr Cys
                85                  90                  95

Ala Asn Ile Val Asn Asp Tyr Trp Gly Gln Gly Thr Gln Val Thr Val
            100                 105                 110

Ser Ser

## Description

### BACKGROUND OF THE INVENTION

### 1. Field of the Invention

The invention relates generally to a method using antibody chain shuffling to create antibodies having inter-species, intra-target cross-reactivity.

### 2. Description of the Related Art

Techniques for designing and producing antibodies, in particular monoclonal antibodies, have been described in the art. Monoclonal antibodies can be designed to selectively bind to a specific antigen. Such antibodies can be used in diagnostic and/or therapeutic methods that make use of the binding affinity and selectivity of the antibody.

U.S. Patent Application Publication 2011/0300140 to Dreier et al. discloses a method for producing Camelid derived conventional antibodies comprising immunizing a Camelid species, such as llama, with an antigen. It has been found that in particular fully outbred Camelid species generate a robust immune response, producing a diverse library of target-specific antibodies characterized by high affinity for the target antigen.

In addition, the conventional Camelid antibodies have been found to possess variable domains having a surprisingly high sequence homology with the corresponding variable domains of human germline antibodies. Moreover, Camelid antibodies have surprisingly high structure similarity to human antibodies. Accordingly Camelid antibodies require few mutations to raise the human character of the variable domains to a level where the level of immunogenicity is acceptably low. These few mutations have little or no effect on the antibody's affinity for the target.

U.S. Patent Application Publication 2011/0165621 to Dreier et al. discloses a protocol for humanizing Camelid derived antibodies.

Several techniques have been developed to mimic the affinity maturation occurring in the immune response of a living animal. Once such techniques is referred to as "chain shuffling;" Chain shuffling was recognized early as a means for generating new antibodies from a library of heavy chains or light chains of antibodies obtained in, for example, a primary immune response. Screening methods can be used to select antibodies obtained in a chain shuffling protocol for affinity and selectivity. In this manner it is possible to generate antibodies that have greater affinity and/or target selectivity than the parent antibodies. See, for example, Kang et al., "Antibody redesign by chain shuffling from random combinatorial immunoglobulin libraries" Proc. Natl. Acad. Sci. USA 88 (1991), 11120-11123. Chain shuffling has also been used for antibody humanization via epitope imprinting.

A large majority of antibodies is being developed for diagnostic and/or therapeutic use in humans. Accordingly, such antibodies are raised and screened for their ability bind to human antigens. For testing of such antibodies in a laboratory environment it is highly desirable that such antibodies also bind to the corresponding antigen present in a non-human animal species, in particular an animal species that is well established in laboratory practice, for example rodents, in particular mouse.

Thus, there is a need for a method for producing antibody molecules having inter-species, intra-target cross-reactivity.

### BRIEF SUMMARY OF THE INVENTION

The present invention addresses this need by providing a method for producing an antibody molecule having inter-species, intra-target cross-reactivity, said method comprising the steps of:
a. creating a first immune library specific for a target antigen from a first species;
b. combining variable regions from the first immune library in a chain shuffling protocol to produce a second immune library;
c. identifying in the second immune library antibody molecules being reactive to the target antigen and to a corresponding antigen from a second species;
wherein the second species is different from the first species.

In a preferred embodiment the first species is man, and the first immune library is created for a human antigen. In this preferred embodiment the second species is a non-human animal, preferably a well-established laboratory animal, such as mouse.

Another aspect of the invention is an antibody molecule having inter-species, intra target cross reactivity.

### DETAILED DESCRIPTION OF THE INVENTION

The following is a detailed description of the invention.

### Definitions

The terms "antibody"; "antigen"; "target antigen"; "specificity"; 'affinity"; "Camelid species"; "VL domain"; "VH domain"; "variable domain"; "L1"; "L2"; "L3"; "H1"; "H2"; "H3"; "framework region"; "constant domain" and "hypervariable loop"; as well as other specific terms as used herein are as defined in U.S. Patent Application Publication No. 2011/0300140 to Dreier et al., the disclosures of which are incorporated herein by reference. The term "antibody molecule" refers to any polypeptide comprising a VH domain and a VL domain which is immunoreactive with and/or exhibits specific binding to a target antigen. Examples include antibodies, immunoglobulins and antibody fragments.

The term "immune library" as used herein refers to a collection of antibody molecules. The antibody molecules may be displayed by a suitable vector, such as phage, plasmid, or phagemid vector. Techniques for creating immune libraries are reviewed in J. Brichta et al., Vet. Med. - Czech, 50, 2005(6): 231-252, the disclosures of which are incorporated herein by reference.

The term "chain shuffling" as used herein refers to a technique for increasing the diversity of an antibody library by providing fragments of antibodies from the library; expressing the fragments in a display tool, for example phage display; and recombining the expressed fragments into antibody molecules. The resulting antibody molecules can be screened for binding to a target antigen X, for the relative absence of undesired binding to a rival antigen Y, or both. Chain shuffling is a commonly used technique for generating antibodies having greater affinity for the target, or greater binding specificity, or both. Chain shuffling can also be used as a humanization protocol for antibodies obtained from a non-human species, such as mouse. Chain shuffling techniques are discussed in Kang et al., "Antibody redesign by chain shuffling from random combinatorial immunoglobulin libraries" Proc. Natl. Acad. Sci. USA 88 (1991), 11120-11123, the disclosures of which are incorporated herein by reference.

Chain shuffling can be carried out with any type of antibody fragment, such as hypervariable loops; complementarity determining regions (CDRs); light chain variable domains; heavy chain variable domains; and the like. For example, a library of light chains may be shuffled with one heavy chain, or a library of heavy chains may be shuffled with one light chain.

The term "cross-reactivity" is used generally to describe non-specific binding between an antibody molecule and an antigen. For example, an antibody X designed to bind antigen X may also exhibit binding to antigen Y. Antibody X would be considered cross-reactive to antigen Y. If antigen X and antigen Y are antigens from the same species this type of cross-reactivity can be referred to as "inter-target, intra-species" cross-reactivity. The term "inter target" refers to the cross-reactivity being to different targets. The term "intra-species" refers to the targets, or antigens, being from the same species. This type of cross-reactivity is generally undesirable, as the unintended binding to antigen Y detracts from, and can interfere with, the desired binding to antigen X. In addition, the unintended binding to antigen Y could interfere with the normal functioning of antigen Y, thus causing undesirable side-effects.

Another form of cross-reactivity exists when an antibody designed to bind to antigen X of a first species (for example human X, or hX) also binds to antigen Y of a second species (for example murine Y, or mY). This would be properly labeled as "inter-target, inter-species cross-reactivity." This type of cross-reactivity may have interesting scientific implications, and could be of use in epitope mapping, for example, but is otherwise not of great value.

Yet another form of cross-reactivity exists when an antibody designed to bind to antigen X of a first species (for example human X, or hX) also binds to the corresponding antigen X of a second species (for example murine X, or mX). This would properly be labeled as "inter-species, intra-target cross-reactivity." This type of cross reactivity is of great practical value, because it permits using the second species (in this example mouse) as an animal model in the study of an antibody being developed for human therapeutic and/or diagnostic use.

In practice, antibodies are screened for and selected on the basis of the desired properties in terms of target affinity and specificity. Whether the selected candidate or candidates has or have desirable inter-species, intra-target cross-reactivity is in essence the luck of the draw. No methods are believed to exist to date to engineer this type of cross-reactivity into an antibody molecule.

It has now been discovered that chain shuffling can be used to produce an antibody molecule having a specific desirable inter-species, intra-target cross-reactivity. Thus, in its most general scope, the present invention relates to a method for producing an antibody molecule having inter-species, intra-target cross-reactivity, said method comprising the steps of:
a. creating a first immune library specific for a target antigen from a first species;
b. combining variable regions from the first immune library in a chain shuffling protocol to produce a second immune library;
c. identifying in the second immune library antibody molecules being reactive to the target antigen and to a corresponding antigen from a second species;
wherein the second species is different from the first species.

Whether an antibody from the second library is reactive to the target antigen and a corresponding antigen from a second species can be established by any suitable method, including direct methods, such as binding assays, or indirect methods testing for a binding-related functionality.

It will be understood that the first species is generally the species for which the antibody molecule is produced. In many cases this species will be human, but in principle the first species can be any species that would benefit from the availability of the antibody molecule, such as husbandry and companion animals, and the like. For the purpose of the present document the first species will assumed to be man, but the reader will keep in mind that this is not intended to be limiting.

The first immune library may be obtained from the first species in any suitable manner. For example, the immune library may be a naive library obtained from one or more healthy volunteers; or it may be obtained from donors who were infected with the target antigen in a natural fashion. If the first species is man, it is generally not ethically possible to create the first immune library from donors that were purposely infected with the target antigen.

In an alternate embodiment the first immune library is obtained from an animal of a third species immunized with the target antigen. The third species can be identical to or different from the first species. If the first species is man, the third species generally is different from the first species. For example, the third species can be a member of the family of *Camelidae,* or Camelids. In a preferred embodiment the third species is llama.

It has been found that Camelids are particularly suitable for producing the first immune library, for a number of reasons. Human target antigens generally provoke a powerful immune response in Camelids. In addition, fully outbred Camelids are readily available for immunization; the use of fully outbred animals contributes to the creation of a highly diverse immune library. As has been reported earlier, the variable domains of antibodies obtained from Camelid species have a high degree of sequence homology with the corresponding human variable domains, as well as a high degree of structural homology. As a consequence such antibodies require little or no mutations for reducing their immunogenicity, so that there is little risk for the high affinity to a human target to be lost.

In particular the high diversity of immune libraries obtained from Camelids significantly increases the probability of successful creation of cross-reactivity by the method of the present invention.

Antibodies for the first immune library are produced and selected in any method known in the art. For example, mature B-cells are harvested from the donor animal; RNA is collected and expressed; antibody fragments are screened for affinity to the target antigen; selected antibodies are sequenced and amplified. Other methods include use of hybridoma technology and cloning; B-cell sorting and transfection; etc.

The first immune library can have any suitable form. For example, the first immune library can be in the form of one or more phage display libraries of variable regions of antibodies from the first immune library. These variable regions can be light chains and heavy chains, for example.

Any chain shuffling protocol can be used for producing the second immune library. It will be understood that, although the purpose of the chain shuffling step of the method is to produce antibody molecules having inter-species, intra-target cross-reactivity, it will be desirable or even necessary to confirm that binding to the target antigen is retained. It may also be desirable to further select antibody molecules from the second immune library for improved selectivity as compared to the antibody molecules of the first immune library. In addition to obtaining the desired intra-target, inter-species cross-reactivity the chain shuffling may also produce antibodies having affinity for intra-species homologues of the target antigen.

Desirably antibodies obtained from the second library have binding affinity to the target antigen that is at least equal to, preferably superior to, the binding affinity to the target antigen exhibited by the most reactive antibody molecules of the first library. For this purpose it may be desirable to use light chain shuffling with a single heavy chain, so that antibody molecules from the second library are likely to bind to the same epitope of the target antigen as do antibody molecules of the first library.

It has also been found that higher affinities are produced in the second library if the light chains and the heavy chain used in a light chain shuffling were obtained from the same individual animal.

An essential step of the method of the invention is identifying in the second immune library antibody molecules having a threshold activity for an antigen from a second species, corresponding to the target antigen from the first species. It is essential also that the second species is different from the third species.

Selection of the second species depends largely on the purpose for which cross-reactivity is sought. In general the usefulness of the inter-species, intra-target cross-reactivity resides in that it offers an avenue for animal testing of the antibody molecule. In many cases it will be desirable to use an animal that is easy to breed and care for, such as rodents, in particular mice. In other cases a deciding factor in the selection of the second species can be the existence of an established disease model in that species, which in many cases is also the mouse. Also, initial toxicity studies are far less expensive in rodents, in particular mouse or rat, than they are in non-human primates, for example. But there may be other factors that require cross-reactivity with a species other than rodents. The essence of the method of the present invention is that it offers antibody molecules having inter-species, intra-target cross-reactivity. How to make the best use of this feature is to be decided on a case-by-case basis.

In an alternate embodiment the second species is a non-human primate, for example cynomolgus (Macaca fascicularis). Non-human primates are more difficult to breed, and more difficult to care for, than are rodents, but non-human primates are evolutionarily much closer to humans, making them oftentimes more suitable for use in the development of antibody molecules for human use.

It will be understood that the method of the invention can also be used for producing antibody molecules that have inter-species, intra-target cross-reactivity for two different species, for example a human antibody molecule for target hX that is cross-reactive to the corresponding antigens in mouse as well as cynomolgus monkey.

In a highly specific embodiment of the invention the first species is man, and the target antigen is human Interleukin 22 receptor (hIL22R). In this highly specific embodiment the second species is a rodent, more specifically mouse, and the corresponding antigen is mouse interleukin 22 receptor (mIL22R). The third species is a member of the *Camelidae* family, for example llama (Lama guanaco).

### DESCRIPTION OF ILLUSTRATIVE EMBODIMENTS/EXAMPLES

Two llamas were immunized with recombinant human IL22R1 (R&D systems, cat 2770-LR). After six weekly injections of recombinant proteins, blood was collected and PBMCs were harvested and their RNA was extracted. After random primed cDNA synthesis the variable regions and the first constant domains of the immunoglobulin heavy and light chain were PCR amplified using specific primers containing extra cloning sites.

The PCR-amplified light chains were digested with ApaLI and AscI while the PCR-amplified VH-CH1 were digested with SfiI and NotI. Both were cloned into the phagemide vector pCB3 vector as described by de Haard et. A1 (JBC 1999) and in in U.S. Patent Application Publication No. 2011/0300140 to Dreier et al.

The diversities of Fab libraries were between 10E8 and 10E9. The phages were produced and phage display selection was done on IL22R. For selection of IL22R-specific clones, IL22R was either coated directly to a MaxiSorp™ plate (Nunc) or captured with a non-competitive anti-human IL22R antibody (MAB2770, R&D systems) or captured with neutravidin after biotinylation. The coating and capture of IL22R was usually done in two different concentrations; for example 5µg/ml and 0.1µg/ml. Phage display selection was done as described in prior art.

After two and three rounds we observed dose-dependent enrichments in all libraries; up to 100-fold after the second round and 10.000-fold after the third round. Remarkably, the enrichments on antibody captured IL22R are clearly higher compared to those on the directly coated IL22R. This could be due to the fact that the IL22R is only 25kDa and the direct coating affects its conformation or limits the available epitope. Therefore a selection campaign was done using biotinylated hIL22R. After selection, single clones are picked up and Fab expressed in the periplasm of the bacteria was tested for their ability to bind to the IL22R and mouse IL22R (R&D system, cat. 4294-MR) and ability to compete IL22 binding.

The VH-CH1 and light chains of clones positive for one or more mentioned characteristic were sequenced. In general, different VH-CH1 were found belonging to different VH families (defined by the CDR3 length and sequence homology). Each VH family was found paired to several light chain families (defined by the frame work subtype, the CDR3 length and sequence homology). The V regions of the most interesting Fab clones were fused to the human constant domains. The DNA encoding for monoclonal antibodies was transfected into mammalian cells to allow production and purification of antibodies. The biological activity of purified antibodies is then tested on cells. Antibodies able to block the IL22R with the highest potency are best candidates for therapeutic antibody.

The development of a therapeutic antibody requires several efficacy and toxicity studies in different animal species before going into man. Therefore, the binding of the antibodies to rodent and particularly mouse IL22R is highly desired to generate *in vivo* proof of concept. The identification of antibody cross-reactive to different distant species, like mouse and humans, is not obvious because of the relatively low sequence identity between the two species (77%). Indeed some antibodies, such as 170B2, are very potent blockers of human IL22R, but have no effect on mouse IL22R. In order to introduce mouse cross-reactivity for 170B2 we used the chain shuffling approach followed by selection on biotinylated mouse IL22R.

For the chain shuffling the VH-CH1 of 170B2 was introduced into the llama light chain library vector obtained from the same llama from which the original 170B2 was obtained. For selection, the human and mouse cytokine receptors were biotinylated and used to select phages that display a Fab cross-reactive for mouse IL22R1. 96-well MaxiSorp™ plate (Nunc) were coated with neutravidin to capture biotinylated IL22R1. Mouse as well as human IL22R1 were captured at two different concentrations for use in the selections. Selection on human IL22R1 was done to determine whether the selection for cross-reactive clones occurred at the expense of binding to human target. The phage libraries were incubated with the captured IL22R1 for two hours before unbound phages were washed away. Bound phages were harvested by trypsine digestion (phage output). Subsequently phage output was rescued and titrated using log phase *E.coli* TG1 cells. Up to three round of selection were done. On mouse IL22R1 we observed large enrichment for the 170B2 Vκ shuffling library: 100-fold after the first round, 1,000-fold after the second round and 10,000-fold after the third round.

After selection, single clones were screened for mouse and human IL22R binding. Positive clones were sequenced, and some were reformatted as monoclonal antibodies for characterization in cell based assay.

**Table 1 containing the VH sequence of the primary clone and the Vkappa sequence of clones selected on mouse IL22R**

| clone | **Vkappa** | **SEQ ID** |
|---|---|---|
| VH_170B2 | EVQLVESGGGLVQPGGSLRLSCAASGFTFSSSWMYWVRQAPGKGLEFVSVINTRSGI THYVESVKGRFTISTDNAKNTLFLQMNSLKSEDTAVYYCATRIQGLIDYWGQGTQVT VSS | No. 1 |
| VK_170B2 | LDIVMTQSPSSLSVSLGDRVTITCQASQSIGSVNALAWYQQKPGQSLKLLIHGASRLQ TGVPSRFSGSGSGTSFTLAISGVEAEDLATYYCLQDYAWPLTFGQGTKVELK | No. 2 |
| VK_198C9 | LDIVMTQSPSSLSASLGDRVTITCQATQSISTFLSWYQQKPGQTPKLLVYGASRLQTG VPSRFSGTGSGTKFTLTISGVEAEDLASYFCLQDYSWPLTFGQGTKVELK | No. 3 |
| VK_197B7 | LDIVMTQSPSSLSASLGDRVTITCQASQSIRSFIDWYQQKPGQAPKLLIYGTSRLETGV PSRFSGTGSGTSFTLTISGVEAEDLATYYCLQDYSWPLTFGQGTKVELK | No. 4 |
| VK_196F8 | LDIQMTQSPSSLSASLGDRVTITCQASQSISSMLAWYQQKPGQAPNLLIYGASRLQTG VPSRFSGSRSGTSFTLTISGVEAEDLATYYCLQDYSWPLTFGQGTKVELK | No. 5 |
| VK_198B7 | LDIQMTQSPSSLSASLGDRVTITCQASQSISSQLAWYQQKPGQAPKLLIYGASRLQTG VPSRFSGSGSGTSFTLTISGVEAEDLATYYCLQDYSWPLTFGQGTKVELK | No. 6 |
| VK_196C2 | LDIQMTQSPSSLSASLGDRVTITCQASQSISSLLAWYQQKPGQAPKLLIYGASRLQTGV PSRFSGSGSGTSFTLTISGVEAEDLATYYCLQDYSWPLTFGQGTKVELK | No. 7 |
| VK_198G11 | LDIVMTQSPSSLSASLGDRVTVTCQASQSIRSSLNWYQQKPGQAPKLLIYGASRLRIGV PSRFSGSASGTSFTLTISGVESEDLATYYCLQDYTWPLSFGSGTRLEIK | No. 8 |
| VK_197D3 | LDIQMTQSPSSLSASLGDRVTVTCQASQSIRSSLNWYQQKPGQAPKLLIYGASRLRIG VPSRFSGSASGTSFTLTISGVEAEDLATYYCLQEYAWPLTFGQGTKVELK | No. 9 |
| VK_197G10 | LDIQMTQSPSSMSASLGDSVTISCQASENIGTQLFWYQQNAGQPPKLLIYGASRLQT GVPSRFSGSGSGTTFTLTISGVETGDLATYYCLQDYSWPLTFGQGTKVELK | No. 10 |

**Table 2 Summary table showing the affinity in biacore and potency in cell based assay**

| **mAb** | **Binding to IL22R1** | | **K_{D} (M) biacore** | | **Potency (pM)** | |
|---|---|---|---|---|---|---|
| | **Hum an** | **Mouse** | **hIL22R** | **mIL22R** | BW-hIL22R | BaF3-mIL22R |
| **170B2** | Yes | No | 1.2E-10 | - | 106 | - |
| **197G10** | Yes | Yes | 2.9E-10 | 4.5E-10 | 124 | 790 |
| **197D3** | Yes | Yes | 8.9E-11 | 3.7E-10 | 57 | + |
| **196C2** | Yes | Yes | 8.0E-11 | 4.5E-10 | 67 | 870 |
| **197B7** | Yes | Yes | 3.8E-11 | 3.9E-10 | 69 | 160 |
| **196F8** | Yes | Yes | 1.0E-11 | 3.1E-10 | 87 | 105 |

### Sequence alignment of the light chain allowing mouse IL22R binding

Clone IC50pM mIL22R

VK_170B2 (~106) DIVMTQSPSSLSVSLGDRVTITCQASQSIGSVNALAWYQQKPGQSLKLLIIGASRLQTGVPSRFSGSGSGTSFTLAISGVEAEDLATYYCLQDYAWPLTFGQGTKVELK
VK_198C9 (83) + DIVMTQSPSSLSASLGDRVTLTCQATQSISTF--LSWYQQKPGQIPKLLVYGASRLQTGVPSRFSGTGSGTKFTLTISGVEAEDLASYFCLQDYSWPLTFGQGTKVELK
VK_197B7 (69) +++ DIVMTQSPSSLSASLGDRVTITCQASQSIRSF--IDWYQQKPGQAPKLLIYGTSRLETGVPSRFSGSGSGTSFTLTISGVEAEDLATYYCLQDYSWPLTFGQGTKVELK
VK_196F8 (87) +++ DIQMTQSPSSLSASLGDRVTITCQASQSISSY--LAWYQQKPGQAPNLLIYGASRLQTGVPSRFSGSRSGTSFTLTISGVEAEDLATYYCLQDYSWPLTFGQGTKVELK
VK_198B7 ND +/- DIQMTQSPSSLSASLGDRVTITCQASQSISSQ--LAWYQQKPGQAPKLLIYGASRLQTGVPSRFSGSGSGTSFTLTISGVEAEDLATYYCLQDYSWPLTFGQGTKVELK
VK_196C2 (67) ++ DIQMTQSPSSLSASLGDRVTITCQASQSISSL--LAWYQQKPGQAPKLLIYGASRLQTGVPSRFSGSGSGTSFTLTISGVEAEDLATYYCLQDYSWPLTFGQGTKVELK
VK_198G11 (132) + DIVMTQSPSSLSASLGDRVTVTCQASQSIRSS LNWYQQKPGQAPKLLIYGASRLRIGVPSRFSGSASGTSFTLTISGVESEDLATYYCLQDY[?]WPLSFGQGTR[?]EIK
VK_197D3 (57) + DIQMTQSPSSLSASLGDRVTVTCQASQSIRSS LNWYQQKPGQAPKLLIYGASRLRIGVPSRFSGSASGTSFTLTISGVEAEDLATYYCLQDYAWPLTFGQGTKVELK
VK_197G10 (124) ++ DIQMTQSPSAMSASLGDSVTISCQASENIGTQ--LFWYQQNAGQPPKLLIYGASRLQTGVPSRFSGSGSGTTFTLTISGVETGDLATYYCLQDYSWPLTFGQGTKVELK

**Alignment of parental VK_170B2 with Vκ shuffled clones which were found to be cross-reactive with mouse IL22R1 and block mIL22R1-hIL22 interaction.** Note the shorter CDR1 of mouse cross-reactive clones compared to parental VK_170B2. Also indicated are the potencies on human (IC50pM) and on mouse IL22R expressing cells. ND, not determined.

In addition to 170B2, the library described in paragraph [0045] contained other clones exhibiting strong binding affinity to human IL22R. Two clones, identified as 169G4 and 158H4, were subjected to VL-chain shuffling and to VH chain shuffling. Of these two clones, 158H4 already had some affinity to mouse IL22R, but this affinity was considered insufficient to be of much practical use. The following table shows the binding characteristics of the shuffled clones:

### Binding characteristics shuffled clones.

**Table 3: Binding characteristics of shuffled clones and their parental clones analyzed on Biacore. No significant binding of 169G4 could be measured to mouse IL22R.**

| | | | Immobilized **human** IL22R ECD | | | | Immobilized **mouse** IL22R ECD | | | |
|---|---|---|---|---|---|---|---|---|---|---|
| clone | VH family | shuffling | Ka (1/Ms) | Kd (1/s) | KD (M) | Chi2 | Ka (1/Ms) | Kd (1/s) | KD (M) | Chi2 |
| 169G4 | 19 | VL | 5.40E+5 | 3.10E-3 | 5.70E-9 | 4.8 | | | | |
| 205A9 | 19 | | 6.70E+5 | 2.80E-4 | 4.20E-10 | 16 | 2.60E+5 | 4.80E-4 | 1.80E-9 | 430 |
| 158H4 | 8 | VH | 1.80E+5 | 3.60E-5 | 2.00E-10 | 1.7 | 1.60E+5 | 1.30E-4 | 8.30E-10 | 170 |
| 205A5 | 8 | | 1.60E+5 | 3.10E-7 | 1.90E-12 | 0.8 | 1.50E+5 | 1.00E-6 | 7.00E-12 | 190 |

The shuffled clones were tested for retention of binding affinity to human, cyno and rhesus IL22R:

### Binding to human, cyno and rhesus IL22R

**Table 4. Species cross reactivity ELISA. ELISA performed by direct coating of 100ng/ml mAb to microsorb plate and binding of biotinylated IL22R ECD, at indicated concentrations. Detection with streptavidin-HRP. Staining with TMB, Absorption at 620nm; absorption values reported in relative absorption units.**

| | | VH family | | | |
|---|---|---|---|---|---|
| | | 19 | 19 | 8 | 8 |
| biotinylated IL22R | | 169G4 | 205A9 | 158H4 | 205A5 |
| human | 100 ng/ml | 1.968 | 2.567 | 2.937 | 2.934 |
| | 2 ng/ml | 1.284 | 1.027 | 0.483 | 1.005 |
| cyno | 100 ng/ml | 2.545 | 2.18 | 2.861 | 2.664 |
| | 2 ng/ml | 2.067 | 9.354 | 1.121 | 1.362 |
| rhesus | 100 ng/ml | 2.452 | 2.59 | 2.679 | 2.769 |
| | 2 ng/ml | 1.978 | 1.966 | 1.289 | 1.174 |
| mouse | 100 ng/ml | 0.078 | 2.358 | 1.724 | 2.36 |
| | 2 ng/ml | 0.084 | 1.863 | 0.201 | 0.172 |

As shown by these data, VL shuffling of 169G4 and selection on mouse IL22R resulted in the identification of clone 205A9, which binds to mouse IL22R with good affinity. Here we see a big improvement for mouse, correct. VH shuffling of 158H4 resulted in identification of clone 205A5, with strongly improved affinity for mouse IL22R. The shuffled clones 205A5 and 205A9 retained the characteristics of their parental clones in terms of epitope (data not shown) and binding to human, cyno and rhesus IL22R.

The relevant chain sequences are as follows:

VL_169G4
LSYELTQPSAVSVLLGQTAKITCQGGSLRSSYAHWYQQKPGQAPVLVITEDDSRPSGI
PERFSGSSSGGTASLTISGAQADDEADYHCQSADISGNPVFGGGTQLTVL

(SEQ ID No. 11)

VL_205A9
HSAVTQPSAVSVSLGQTARITCQGGNFGSYYASWYQQKPGQAPVLVIYDDDRRPSGI
PERFSGSSSGGRSTLTISGAQSGDEGDYYCQSADLSGNPVFGGGTHLTVL

(SEQ ID No. 12)

VH_205A5
QVQLVESGGGLVQPGGSLRLSCAASGFTFNSYEMYWVRQAPGKGLEWVSGITADG
GYTYYADSVKGRFTMSRDNAKNTLYLQMNSLKPEDTALYYCANLWNDYWGQGTQ
VIVSS

(SEQ ID No. 13)

VH_158H4

QLQVVESGGGLVQPGGSLSLSCATSGFTFSNYEMNWVRQAPGKGFEWVSLINSGGS

YTRYTDSVKGRFTISRDNAKKTLYLQMNSLKPEDTAVYYCANIVNDYWGQGTQVT

VSS

(SEQ ID No. 14)

Thus, the invention has been described by reference to certain embodiments discussed above. It will be recognized that these embodiments are susceptible to various modifications and alternative forms well known to those of skill in the art. For example, the method may be modified by using a different platform for creating the first immune library; by using a different method for creating the first immune library; by using a different chain shuffling protocol; and the like.

Many modifications in addition to those described above may be made to the structures and techniques described herein without departing from the spirit and scope of the invention. Accordingly, although specific embodiments have been described, these are examples only and are not limiting upon the scope of the invention.

The invention can also be understood with reference to the following clauses:
1. A method for producing an antibody molecule having inter-species, intra-target cross-reactivity, said method comprising the steps of:
   a. creating a first immune library specific for a target antigen from a first species;
   b. combining variable regions from the first immune library in a chain shuffling protocol to produce a second immune library;
   c. identifying in the second immune library antibody molecules being reactive to the target antigen and to a corresponding antigen from a second species;
      wherein the second species is different from the first species.
2. The method of clause 1 wherein the variable regions used in the chain shuffling protocol of step b. are obtained from one individual animal.
3. The method of clause 1 wherein the first immune library is obtained from a member of the first species infected with the target antigen.
4. The method of clause 1 wherein the first immune library is obtained from an animal of a third species immunized with the target antigen.
5. The method of clause 4 wherein the first species and the third species are identical.
6. The method of clause 4 wherein the first species and the third species are different.
7. The method of any one of the preceding clauses wherein the first species is man.
8. The method of clause 6 or 7 wherein the third species belongs to *Camelidae.*
9. The method of clause 8 wherein the third species is llama.
10. The method of any one of the preceding clauses wherein the second species is a rodent.
11. The method of clause 10 wherein the second species is mouse.
12. The method of any one of clauses 1 through 9 wherein the second species is a non-human primate.
13. The method of clause 12 wherein the second species is *Cynomolgus.*
14. The method of any one of the preceding clauses wherein the first species is man and the target antigen is human IL22R.
15. The method of clause 14 wherein the third species belongs to *Camelidae.*
16. The method of clause 15 wherein the second species is a rodent.
17. The method of clause 16 wherein the second species is mouse.
18. An antibody molecule obtained by the method of clause 1.
19. The antibody molecule of clause 18 which is reactive to human IL22R and to a non-human IL22R.
20. An antibody according to clause 18 having a VH sequence that is at least 90% homologous to SEQ. ID. No. 1, 13 or 14.
21. An antibody according to clause 18 having a VL sequence that is at least 90% homologous to SEQ. ID. No. 11 or 12.
22. An antibody according to clause 18 having a Vkappa sequence that is at least 90% homologous to at least one of SEQ. ID, 2, 3, 4, 5, 6, 7, 8, 9, or 10.
23. An antibody that competes in binding to human IL22R with the antibody of clause 20, 21 or 22.
24. An antibody that competes in binding to murine IL22R with the antibody of clause 20, 21 or 22.

## Claims

1. An antibody molecule obtained by a method for producing an antibody molecule having inter-species, intra-target cross-reactivity, said method comprising the steps of:
a. creating a first immune library specific for a target antigen from a first species;
b. combining variable regions from the first immune library in a chain shuffling protocol to produce a second immune library;
c. identifying in the second immune library antibody molecules being reactive to the target antigen and to a corresponding antigen from a second species;
wherein the second species is different from the first species.

2. The antibody molecule of claim 1 which is reactive to human IL22R and to a non-human IL22R.

3. An antibody according to claim 1 having a VH sequence that is at least 90% homologous to SEQ. ID. No. 1, 13 or 14.

4. An antibody according to claim 1 having a VL sequence that is at least 90% homologous to SEQ. ID. No. 11 or 12.

5. An antibody according to claim 1 having a Vkappa sequence that is at least 90% homologous to at least one of SEQ. ID, 2, 3, 4, 5, 6, 7, 8, 9, or 10.

6. An antibody that competes in binding to human IL22R with the antibody of claim 3, 4 or 5.

7. An antibody that competes in binding to murine IL22R with the antibody of claim 3, 4 or 5.
